# EUROPEAN PATENT SPECIFICATION

(11) **EP 4 367 101 B1**
(45) Date of publication and mention of the grant of the patent: **30.09.2026**
(21) Application number: 22738447.6
(22) Date of filing: 28.06.2022
(51) Int. Cl.: C07D 231/14, C07C 251/40, C07D 271/06

(54) **PREPARATION OF SUBSTITUTED AMIDOXIMES**
HERSTELLUNG VON SUBSTITUIERTEN AMIDOXIMEN
PRÉPARATION D'AMIDOXIMES SUBSTITUÉES

(30) Priority: 08.07.2021 IN 202121030667; 30.09.2021 EP 21200185
(43) Date of publication of application: 15.05.2024
(73) Proprietor: BASF Agricultural Solutions Deutschland GmbH, 67117 Limburgerhof (DE)
(72) Inventor: GEBHARDT, Joachim, 67056 Ludwigshafen (DE); GARIVET, Guillaume Michel Jacques, 67056 Ludwigshafen (DE); BORATE, Kailaskumar, Navi Mumbai 400705 (IN); KNOLL, Daniel Maximilian, 67056 Ludwigshafen (DE); GOETZ, Roland, 67056 Ludwigshafen (DE)
(74) Representative: AP-IP
(86) International application number: PCT/EP2022/067677
(87) International publication number: WO 2023/280630

(56) References cited:
- WO-A1-2010/080357
- WO-A1-2015/185485
- VÖRÖS ATTILA ET AL: "An experimental and theoretical study of reaction mechanisms between nitriles and hydroxylamine - Supplementary informations", ORGANIC & BIOMOLECULAR CHEMISTRY, vol. 12, no. 40, 1 January 2014 (2014-01-01), pages 1 - 10, XP093246998, ISSN: 1477-0520, DOI: 10.1039/c4ob00854e
- VÖRÖS ATTILA ET AL: "An experimental and theoretical study of reaction mechanisms between nitriles and hydroxylamine", ORGANIC & BIOMOLECULAR CHEMISTRY, vol. 12, no. 40, 1 January 2014 (2014-01-01), pages 8036 - 8047, XP055892419, ISSN: 1477-0520, DOI: 10.1039/C4OB00854E

## Description

The present invention relates to a process for the preparation of substituted amidoximes of formula I, which can be obtained through reaction of nitrile compounds of formula II with free hydroxylamine in a solvent.

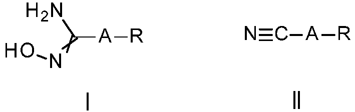

Amidoximes I are versatile synthesis intermediates, which can be converted to substituted 3-aryl-5-trifluoromethyl-1,2,4-oxadiazoles that are known to be useful for controlling phytopathogenic fungi, for example from WO 2015/185485 A1 and WO 2017/211649 A1.

Prior art references often suggest using a large excess of hydroxylamine in the form of one of its acid addition salts, for example hydroxylamine hydrochloride, hydroxylammonium acetate, or hydroxylammonium sulfate for the preparation of amidoximes I from nitriles II. These salts are typically employed in syntheses on laboratory scale because they can be handled easily and safely. Hydroxylamine as a free base will readily decompose if not properly stored and handled. Uncontrolled decomposition is rapid and can be violent, generating heat and large quantities of gases that may increase the pressure. This potential increase in pressure can cause the container to rupture with a subsequent release. Dangerous explosive-like decompositions can be caused by overheating, contamination, and/or by concentrating the solution.

It was reported that free hydroxylamine may be generated *in situ* by addition of an auxiliary base to the corresponding acid addition salts. Suitable auxiliary bases can be of organic or inorganic nature, for example, amines, pyridines, carbonates, hydrogencarbonates, hydroxides, or alcoholates. These transformations may also provide satisfactory yields when using approximately equimolar amounts of the hydroxylamine salt based on the amount of nitrile. The following references give representative examples of procedures that form part of the prior art: WO 2015/185485 A1 (working example, step 1) describes the preparation of amidoximes using hydroxylamine hydrochloride and sodium bicarbonate in ethanol at reflux. WO 2017/211649 A1 (working example I.1) likewise discloses the preparation of amidoximes using hydroxylamine hydrochloride and potassium carbonate in a mixture of ethanol and water at reflux. WO 2010/080357 A1 (working examples, method I, step 2) describes the preparation of amidoximes using an aqueous solution of free hydroxylamine in ethanol as solvent at 80°C.

From an economic point of view it is desirable in industrial large-scale operations dealing with the production of amidoximes I to minimize both, the amount of solvent used in the process and the amount of hydroxylamine. The use of free hydroxylamine is particularly attractive because it i) is more reactive than its salts and provides high yields even if employed in equimolar amounts, ii) is cheaper and more atom-efficient than its salts, iii) does not require the use of a base, iv) leads to significantly smaller waste streams, i.e. without salts, and v) avoids handling of solid material in the process.

When using minimal amounts of a solvent in the process according to the present invention amidoximes of formula I and nitriles of formula II have limited solubility. Thus, at the start of the reaction, the reaction mixture is a suspension containing particulate nitrile II. The inventors found that, in reactions where the mixture obtained in step 1 is a suspension containing particulate nitrile II, the total amount of solid material in the suspension increases over time in step 2, i.e. as the conversion of the nitrile II progresses, which is arguably due to the precipitation of amidoxime I. This build-up of solid material causes a significant increase of viscosity of the reaction mixture to the point where proper agitation of the reaction mixture eventually gets very hard or is no longer possible resulting in the complete blockage of the reactor. Excessively high levels of viscosity have detrimental effects on the conversion rate and lead to the formation of unwanted side-products.

In view of this, it was an object of the present invention to overcome these disadvantages and to provide an improved and more economical process, which enables the preparation of amidoximes I on an industrial scale in high yield and with low amounts of side-products.

The inventors found that the slow addition of free hydroxylamine to suspensions containing nitrile II surprisingly alleviated the effect of amidoxime precipitation, thus keeping the viscosity of the reaction mixture, technically speaking, at a justifiable level. In this process almost quantitative yields of the desired amidoximes I were obtained.

Hence, the process of the present invention is more cost efficient and eco-friendly than previously reported processes as it employs readily available and cheap reactants in the smallest possible amounts. Furthermore, the present invention demonstrates that fast conversion of the starting material is achieved at comparatively low reaction temperatures.

Accordingly, the present invention relates to a process for preparing amidoximes of formula I, 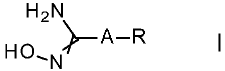 wherein
- A: is phenyl or a 5- or 6-membered aromatic heterocycle; wherein the ring member atoms of the aromatic heterocycle include besides carbon atoms 1, 2, 3, or 4 heteroatoms selected from N, O, and S as ring member atoms with the provision that the heterocycle cannot contain 2 contiguous atoms selected from O and S; and wherein A is further unsubstituted or further substituted with additional n identical or different radicals R^{A}; wherein
n is 0,1, 2, 3, or 4;
R^{A} is independently selected from the group consisting of halogen, cyano, C₁-C₆-alkyl, C₁-C₆-haloalkyl, C₁-C₆-alkoxy, and C₁-C₆-haloalkoxy;
- R: is methyl, chloromethyl, hydroxymethyl, trichloromethyl, ethyl, iso-propyl, OH, SH, cyano, halogen, CH₂F, CHF₂, 2,2,2-trifluoroethyl, cyclopropyl, -C(=O)H, -C(=NOR²)H, -C(=O)OH, -C(=O)OR¹, -C(=W)N(R¹R²), -CR³R⁴-N(R¹R²), -CR³R⁴-OR¹, -C(=NR¹)R³, -C(=O)R³, -CR³R⁴-C(=O)OH, -CR³R⁴-C(=O)R¹, -CR³R⁴-C(=W)N(R¹R²), -O-CR³R⁴-C(=O)OH, -O-CR³R⁴-C(=O)R¹, -O-CR³R⁴C(=W)N(R¹R²), -CR³R⁴-N(R²)-C(=W)R¹, -CR³R⁴-S(=O)₂R¹, or -CR³R⁴-N(R²)-S(=O)₂R¹; wherein
- W: is O or S;
- R²: is hydrogen, C₁-C₆-alkyl, C₂-C₆-alkenyl, C₂-C₆-alkynyl, C₁-C₆-alkoxy, C₃-C₁₁-cycloalkyl, -C(=O)-C₁-C₆-alkyl, -C(=O)-C₃-C₁₁-cycloalkyl, or -C(=O)-O-C₁-C₆-alkyl; and wherein any of the aliphatic or cyclic groups in R² are unsubstituted or substituted with 1, 2, 3, or up to the maximum possible number of identical or different radicals selected from the group consisting of halogen, hydroxy, oxo, cyano, C₁-C₆-alkyl, C₁-C₆-alkoxy, and C₃-C₁₁-cycloalkyl:
- R¹: is C₁-C₆-alkyl, C₁-C₆-alkoxy, C₃-C₁₁-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₆-alkylamino, diC₁-C₆-alkylamino, -C(=O)-C₁-C₆-alkyl, -C(=O)-O-C₁-C₆-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl, or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O, and S as ring member atoms with the provision that the heterocycle cannot contain 2 contiguous atoms selected from O and S; and wherein any of the above-mentioned aliphatic or cyclic groups are unsubstituted or substituted with 1, 2, 3, or up to the maximum possible number of identical or different groups R^{1a}; or
- R¹ and R²,: together with the nitrogen atom to which they are attached, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O, and S as ring member atoms with the provision that the heterocycle cannot contain 2 contiguous atoms selected from O and S; and wherein the heterocycle is unsubstituted or substituted with 1, 2, 3, 4, or up to the maximum possible number of identical or different groups R^{1a}; wherein
R^{1a} is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, -C(=O)-C₁-C₄-alkyl, -C(=O)-O-C₁-C₄-alkyl, C₁-C₆-alkylsulfonyl, hydroxyC₁-C₄-alkyl, -C(=O)-NH₂, -C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl, or C₁-C₄-alkoxy-C₁-C₄-alkyl;
- R³, R⁴: independently of each other are selected from the group consisting of hydrogen, halogen, cyano, C₁-C₄-alkyl, C₁-C₄-alkenyl, C₁-C₄-alkynyl, C₁-C₄-haloalkyl and C₁-C₄-alkoxy; or
- R³ and R⁴: together with the carbon atom to which they are bound form a cyclopropyl group,
by reacting a nitrile of formula II, wherein the variables A and R are as defined above for compounds of formula I,

N≡C-A-R II

with free hydroxylamine in a solvent comprising an alkyl alcohol of formula III

C₁-C₆-alkyl-OH III,

the process comprising the following steps:
step 1: charging a reaction vessel with a solvent and nitrile II;
step 2: metering 0.9 to 1.5 equivalents of free hydroxylamine, based on the amount of nitrile II, into the mixture obtained in step 1;
whereas the process is characterized in that the mixture obtained in step 1 is a suspension containing particulate nitrile II; the free hydroxylamine in step 2 is metered to nitrile II over a period of equal to or more than 15 minutes; and wherein the temperature of the reaction mixture in step 2 of the process is between 20°C to 60°C.

The general expression "compound I" as used herein is equivalent to the expression "compounds of formula I". Accordingly, for example, the expression "nitrile of formula II" as used herein is equivalent to the expression "nitrile II".

The term "free hydroxylamine" as used herein shall mean hydroxylamine (HO-NH₂) in its form as a free base, i.e. in its non-protonated form; whereas "hydroxylammonium salts" are formed with hydroxylamine in the presence of, or upon addition of, an acid, for example, but not limited to, hydrogen chloride, sulfuric acid, or acetic acid.

Since nitrile II may contain small amounts of residual acid from previous synthesis steps, a small amount of hydroxylammonium salts may be present in the process of the present invention. Accordingly, in one aspect of the invention the reaction mixture obtained in step 1 of the process contains less than 0.2 equivalents of hydroxylammonium salts after the addition of the free hydroxylamine to nitrile II, based on the total amount of hydroxylamine in the reaction mixture.

In another aspect of the present invention the reaction mixture obtained in step 1 of the process contains less than 0.1 equivalents of hydroxylammonium salts after the addition of the free hydroxylamine to nitrile II, based on the amount of nitrile II in the reaction mixture.

In a further aspect of the present invention the reaction mixture obtained in step 1 of the process contains less than 0.05 equivalents of hydroxylammonium salts after the addition of the free hydroxylamine to nitrile II, based on the amount of nitrile II in the reaction mixture.

In one embodiment of the present invention 0.9 to 1.5 equivalents of free hydroxylamine are added in step 2 to the reaction mixture obtained in step 1.

In one embodiment of the present invention 1.0 to 1.5 equivalents of free hydroxylamine are added in step 2 to the reaction mixture obtained in step 1.

In another embodiment of the present invention 1.0 to 1.2 equivalents of free hydroxylamine are added in step 2 to the reaction mixture obtained in step 1.

Typically, hydroxylamine is not used in pure form for safety reasons but as an aqueous solution with a concentration of up to 50% (w/w).

In one aspect of the present invention the free hydroxylamine in step 2 is metered to nitrile II over a period of equal to or more than 30 minutes.

In one aspect of the present invention the free hydroxylamine in step 2 is metered to nitrile II over a period of equal to or more than 1 hour.

In one aspect of the present invention the free hydroxylamine in step 2 is metered to nitrile II over a period of equal to or more than 2 hours.

In one aspect of the present invention the free hydroxylamine in step 2 is metered to nitrile II over a period of equal to or more than 3 hours.

In one aspect of the present invention the free hydroxylamine in step 2 is metered to nitrile II over a period of 15 minutes to 48 hours.

In a further aspect of the present invention the free hydroxylamine in step 2 is metered to nitrile II over a period of 15 minutes to 10 hours.

In another aspect of the present invention the free hydroxylamine in step 2 is metered to nitrile II over a period of 15 minutes to 5 hours.

In another aspect of the present invention the free hydroxylamine in step 2 is metered to nitrile II over a period of 30 minutes to 5 hours.

After the addition of the free hydroxylamine the reaction mixture is stirred until the conversion of nitrile II is complete. This process may take between 1 to 24 hours.

In another embodiment the temperature of the reaction mixture in step 2 of the process is between 20°C and 60°C.

In a preferred embodiment the temperature of the reaction mixture in step 2 of the process is between 30°C and 50°C. At temperatures below 50°C the formation of side-products is sufficiently suppressed.

In a more preferred embodiment the temperature of the reaction mixture in step 2 of the process is between 35°C and 45°C.

In one aspect of the process the mixture obtained in step 1 and as used in step 2 is a suspension containing particulate nitrile II.

In one embodiment the mixture obtained in step 1 contains equal to or more than 350 g of nitrile II per kilogram of solvent.

In one embodiment the mixture obtained in step 1 contains equal to or more than 450 g of nitrile II per kilogram of solvent.

In another embodiment the mixture obtained in step 1 contains equal to or more than 550 g of nitrile II per kilogram of solvent.

In one aspect of the invention the process is carried out in the absence of an auxiliary base. The term "auxiliary base" as used herein refers to a base, which does not take part in the reaction as a reactant but acts as a scavenger for protons to generate free hydroxylamine from hydroxylammonium salts.

The term "solvent" herein refers to an inert solvent, i.e. a solvent, which is not taking part in the reaction. This means that the auxiliary solvent is not identical with the reactants.

In one aspect of the present invention the process is conducted in a solvent selected from a dipolar organic solvent or water, or mixtures of dipolar organic solvents and water. Suitable dipolar organic solvents are, for example, ethers (diethylether, dibutylether, *tert-*butylmethylether, ethylene glycol dimethyl ether, ethylene glycol, diethyl ether, diethylene glycol dimethyl ether, 2-methyltetrahydrofuran, tetrahydrofuran, dioxane, diethylene, glycol monomethyl- or monoethyl ether), *N*-substituted lactams (*N*-methylpyrrolidone), carboxamides (*N*,*N*-dimethylformamide, *N*,*N*-dimethylacetamide), acyclic ureas (dimethyl imidazolinum), sulphoxides, sulphones (dimethyl sulfoxide, dimethyl sulfone, tetramethylene sulfoxide, tetramethylene sulfone), and alkyl alcohols (methanol, ethanol, *n*-propanol, *iso*-propanol, *n-*butanol, *iso*-butanol, *sec*-butanol, *tert*-butanol, 1-pentanol, 1-hexanol, 2-ethylhexanol, 3-methyl-1-butanol, cyclohexanol).

In a preferred embodiment the solvent comprises an alkyl alcohol of formula III, or mixtures thereof,

C₁-C₆-alkyl-OH III.

In one embodiment of the present invention, the solvent in the process comprises methanol, ethanol, *n*-propanol, *iso*-propanol, *n*-butanol, *iso*-butanol, *sec*-butanol, or *tert*-butanol.

In a preferred embodiment the solvent comprises methanol or ethanol.

In another particularly preferred embodiment the solvent is methanol or mixtures of methanol with water. In a particularly preferred embodiment the solvent is methanol.

In a particularly preferred embodiment the solvent is ethanol or mixtures of ethanol with water. In a particularly preferred embodiment the solvent is ethanol.

In a particularly preferred embodiment the process is conducted in a solvent comprising an alkyl alcohol of formula III, more particularly methanol, ethanol, *n*-propanol, *iso*-propanol, *n*-butanol, *iso*-butanol, *sec*-butanol, or *tert*-butanol; wherein 0.9 to 1.5 equivalents of free hydroxylamine is metered in step 2 to the reaction mixture obtained in step 1 over a period of equal to or more than 15 minutes; and wherein the temperature of the reaction mixture in step 2 of the process is between 20°C to 60°C.

In a particularly preferred embodiment the process is conducted in a solvent comprising an alkyl alcohol of formula III, more particularly methanol, ethanol, *n*-propanol, *iso*-propanol, *n*-butanol, *iso*-butanol, *sec*-butanol, or *tert*-butanol; wherein the mixture obtained in step 1 is a suspension containing particulate nitrile II; wherein 0.9 to 1.5 equivalents of free hydroxylamine is metered in step 2 to the reaction mixture obtained in step 1 over a period of equal to or more than 15 minutes; and wherein the temperature of the reaction mixture in step 2 of the process is between 20°C to 60°C.

In a particularly preferred embodiment the process is conducted in a solvent comprising an alkyl alcohol of formula III, more particularly methanol, ethanol, *n*-propanol, *iso*-propanol, *n*-butanol, *iso*-butanol, *sec*-butanol, or *tert*-butanol; wherein the mixture obtained in step 1 contains equal to or more than 350 g of nitrile II per kilogram of solvent; wherein 0.9 to 1.5 equivalents of free hydroxylamine is metered in step 2 to the reaction mixture obtained in step 1 over a period of equal to or more than 15 minutes; and wherein the temperature of the reaction mixture in step 2 of the process is between 20°C to 60°C.

In a further particularly preferred embodiment the process is conducted in a solvent comprising methanol or ethanol; wherein 0.9 to 1.5 equivalents of free hydroxylamine is metered in step 2 to the reaction mixture obtained in step 1 over a period of equal to or more than 15 minutes; and wherein the temperature of the reaction mixture in step 2 of the process is between 20°C to 60°C.

In a further particularly preferred embodiment the process is conducted in a solvent comprising methanol or ethanol; wherein the mixture obtained in step 1 is a suspension containing particulate nitrile II; wherein 0.9 to 1.5 equivalents of free hydroxylamine is metered in step 2 to the reaction mixture obtained in step 1 over a period of equal to or more than 15 minutes; and wherein the temperature of the reaction mixture in step 2 of the process is between 20°C to 60°C.

In a further particularly preferred embodiment the process is conducted in a solvent comprising methanol or ethanol; wherein the mixture obtained in step 1 is a suspension containing particulate nitrile II; wherein 0.9 to 1.5 equivalents of free hydroxylamine is metered in step 2 to the reaction mixture obtained in step 1 over a period of equal to or more than 30 minutes; and wherein the temperature of the reaction mixture in step 2 of the process is between 30°C to 50°C.

In a further particularly preferred embodiment the process is conducted in a solvent comprising methanol or ethanol; wherein the mixture obtained in step 1 contains equal to or more than 350 g of nitrile II per kilogram of solvent; wherein 0.9 to 1.5 equivalents of free hydroxylamine is metered in step 2 to the reaction mixture obtained in step 1 over a period of equal to or more than 15 minutes; and wherein the temperature of the reaction mixture in step 2 of the process is between 20°C to 60°C.

In a further particularly preferred embodiment the process is conducted in a solvent comprising methanol or ethanol; wherein the mixture obtained in step 1 contains equal to or more than 350 g of nitrile II per kilogram of solvent; wherein 0.9 to 1.5 equivalents of free hydroxylamine is metered in step 2 to the reaction mixture obtained in step 1 over a period of equal to or more than 30 minutes; and wherein the temperature of the reaction mixture in step 2 of the process is between 30°C to 50°C.

In still another preferred embodiment the process is conducted in a solvent comprising methanol or ethanol; wherein the mixture obtained in step 1 contains equal to or more than 450 g of nitrile II per kilogram of solvent; wherein 1.0 to 1.5 equivalents of free hydroxylamine is metered in step 2 to the reaction mixture obtained in step 1 over a period of equal to or more than 30 minutes; and wherein the temperature of the reaction mixture in step 2 of the process is between 30°C to 50°C.

The process of the present invention is typically carried out at atmospheric pressure. After the addition of the free hydroxylamine is complete, water is slowly added to the reaction mixture in order to reduce product solubility for work up. The reaction mixture is then cooled to 0-5°C before filtration in order to avoid product losses in the mother liquor.

In one aspect of the present invention the variable A is phenyl in compounds of formula I and II. In one embodiment of the present invention radical R^{A} in compounds of formula I and II is halogen, C₁-C₆-alkyl, C₁-C₆-haloalkyl, C₁-C₆-alkoxy, or C₁-C₆-haloalkoxy; particularly fluorine.

In one aspect n is 1 and R^{A} is fluorine in compounds of formula I and II.

In a preferred embodiment the variable n is 0 in compounds of formula I and II.

In one aspect the present invention relates to a process as defined above, wherein the nitrile is of formula Il.b, 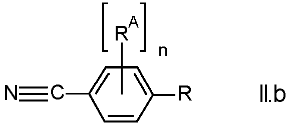 wherein n is 0 or 1; and wherein the meaning of R is as defined or preferably defined herein for compounds of formula I; and wherein R^{A} is selected from the group consisting of halogen, C₁-C₆-alkyl, C₁-C₆-haloalkyl, C₁-C₆-alkoxy, and C₁-C₆-haloalkoxy; to obtain amidoximes of formula I.b, 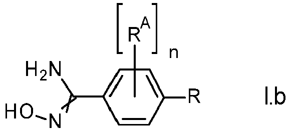 wherein the variables n, R^{A}, and R have the meaning as defined for compounds Il.b.

In another embodiment n is 1 and R^{A} is fluorine in compounds of formula I.b and II.b.

In a preferred embodiment n is 0 in compounds of formula I.b and II.b.

In one embodiment the variables in compounds of formula I, II, I.b, and Il.b have the following meaning:
- R^{A}: is fluorine;
- n: is 0 or 1;
- R: is methyl, chloromethyl, hydroxymethyl, trichloromethyl, , - C(=O)H, -C(=NOR²)H, -C(=O)OH, OH, SH, cyano, halogen, -C(=O)NR¹R², -CH₂-N(R²)-C(=O)R¹, -CH₂-N(R²)-S(=O)₂R¹, 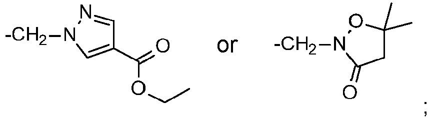
- R¹: is methyl, ethyl, *n*-propyl, *iso*-propyl, *n*-butyl, *sec*-butyl, *iso*-butyl, cyclopropyl, 2-methoxyiminoethyl, bicyclo[1.1.1]pentan-1-yl, or phenyl; and wherein the phenyl group is unsubstituted or substituted with 1, 2, 3 or up to the maximum possible number of identical or different radicals selected from the group consisting of fluorine, chlorine, cyano, methyl, ethyl, methoxy, trifluoromethyl, trifluoromethoxy, difluoromethyl, difluoromethoxy, and cyclopropyl;
- R²: is hydrogen, methyl, ethyl, methoxy, ethoxy, or cyclopropyl.

In a further embodiment the variables in compounds of formula I, II, I.b, and Il.b have the following meaning:
- R^{A}: is fluorine;
- n: is 0 or 1;
- R: is methyl, -C(=O)OH, -C(=O)NR¹R², -CH₂-N(R²)-C(=O)R¹, -CH₂-N(R²)-S(=O)₂R¹, 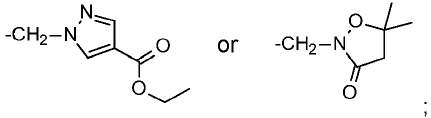
- R¹: is C₁-C₆-alkly, phenyl, or cyclopropyl, wherein the phenyl ring is unsubstituted or substituted with 1, 2, 3, or 4 identical or different groups selected from halogen;
- R²: is hydrogen, methyl, ethyl, methoxy, ethoxy, or cyclopropyl.

In yet another embodiment the variables in compounds of formula I, II, I.b, and Il.b have the following meaning:
- R^{A}: is fluorine;
- n: is 0 or 1;
- R: is -CH₂-N(R²)-C(=O)R¹, -CH₂-N(R²)-S(=O)₂R¹, 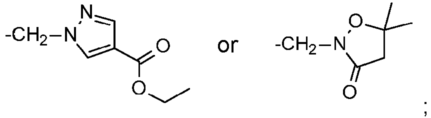
- R¹: is C₁-C₆-alkly, or cyclopropyl;
- R²: is hydrogen, methyl, methoxy, ethoxy, or cyclopropyl.

In another embodiment the variables in compounds of formula I, II, I.b, and Il.b have the following meaning:
- R^{A}: is fluorine;
- n: is 0 or 1;
- R: is methyl, -C(=O)OH, or -C(=O)NR¹R²;
- R¹: is methyl or phenyl, wherein the phenyl ring is unsubstituted or substituted with 1, 2, 3, or 4 identical or different groups selected from halogen;
- R²: is hydrogen, methyl, ethyl, methoxy, or ethoxy.

In still another embodiment the variables in compounds of formula I, II, I.b, and Il.b have the following meaning:
- n: is 0;
- R: is -C(=O)NR¹R²;
- R¹: is methyl, 2-methoxyiminoethyl, bicyclo[1.1.1]pentan-1-yl, 2-fluoro-phenyl, 4-fluoro-phenyl, or 2,4-difluorophenyl; in particular methyl or 2-fluoro-phenyl;
- R²: is hydrogen.

In a particularly advantageous approach a compound of formula I.b, wherein n is 0, may be reacted with an activated form of trifluoroacetic acid (for example using trifluoro acetic acid anhydride, trifluoroacetic acid halides, or using methyl or ethyl trifluoroacetate, for reference see WO 2015/185485 A1, WO 2019/02045 A1, PCT/EP2021/052256 and WO 2020/212513 A1) to obtain the compound of formula IV, 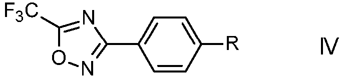 wherein the variable R has the meaning as defined or preferably defined herein for compounds I.b. This two-step transformation is particularly preferred in regard to compounds IV and I.b, wherein R is -C(=O)N(R¹R²), R¹ is hydrogen, and R² is 2-fluorophenyl.

In a further embodiment of the invention a compound of formula I.b, wherein R is methyl, is converted into valuable chemical products or intermediates. Accordingly, in one embodiment, compounds of formula I.b, wherein n is 0 and R is methyl, can be further chlorinated to obtain a compound of formula IV.a.

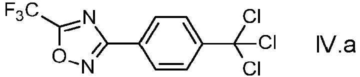

The chlorination of the methyl group R of compounds of formula I.b can be achieved as described in WO 2019/020451 A1 and the references cited therein.

In a further embodiment the compound of formula IV.a is hydrolyzed to obtain a compound of formula V.

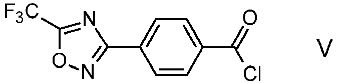

In one embodiment this transformation is carried out in the presence of catalytic amounts of a Lewis acid and water to obtain a compound of formula V, as described in WO 2019/020451 A1 and the references cited therein. Preferably, the Lewis acid is a metal salt, for example aluminum(III) chloride or iron(III) chloride, particularly iron(III) chloride.

In another embodiment the compound of formula I.b, wherein n is 0 and R is -C(=O)OH, is chlorinated to obtain a compound of formula V.

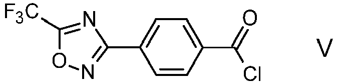

These transformations are described in WO 2019/020451 A1 and WO 2017/211649 A1 and the references cited therein.

In one embodiment, the compound of formula V is reacted with an amine of formula VI,

R¹-NH-R² VI

wherein
- R¹: is C₁-C₆-alkyl, C₁-C₆-alkoxy, C₃-C₁₁-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₆-alkylamino, diC₁-C₆-alkylamino, -C(=O)-C₁-C₆-alkyl, -C(=O)-O-C₁-C₆-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl, or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O, and S as ring member atoms with the provision that the heterocycle cannot contain 2 contiguous atoms selected from O and S; and wherein any of the above-mentioned aliphatic or cyclic groups are unsubstituted or substituted with 1, 2, 3, or up to the maximum possible number of identical or different groups R^{1a}; or
- R¹ and R²,: together with the nitrogen atom to which they are attached, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O, and S as ring member atoms with the provision that the heterocycle cannot contain 2 contiguous atoms selected from O and S; and wherein the heterocycle is unsubstituted or substituted with 1, 2, 3, 4, or up to the maximum possible number of identical or different groups R^{1a}; wherein
R^{1a} is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, (C=O)-C₁-C₆-alkyl, C(=O)-O-C₁-C₆-alkyl, C₁-C₆-alkylsulfonyl, hydroxyC₁-C₄-alkyl, C(=O)-NH₂, C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl, or C₁-C₄-alkoxy-C₁-C₄-alkyl;
- R²: is hydrogen, C₁-C₆-alkyl, C₂-C₆-alkenyl, C₂-C₆-alkynyl, C₁-C₆-alkoxy, C₃-C₁₁-cycloalkyl,-C(=O)H, -C(=O)-C₁-C₆-alkyl, -C(=O)-C₃-C₁₁-cycloalkyl, or -C(=O)-O-C₁-C₆-alkyl; and wherein any of the aliphatic or cyclic groups in R² are unsubstituted or substituted with 1, 2, 3, or up to the maximum possible number of identical or different radicals selected from the group consisting of halogen, hydroxy, oxo, cyano, C₁-C₆-alkyl, C₁-C₆-alkoxy, and C₃-C₁₁-cycloalkyl;
to obtain a compound of formula VII.

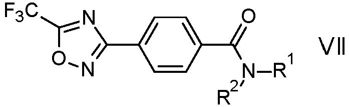

These transformations are also described in WO 2019/020451 A1 and WO 2017/211652 A1 and the references cited therein.

In another embodiment, the compound of formula VII is used to obtain a compound of formula VIII, 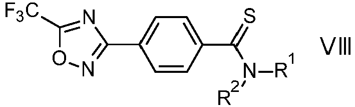 as described in WO 2019/020451 A1 and WO 2017/211649 A1 and the references cited therein.

In a preferred embodiment the variables R¹ and R² in compounds of formula I, I.b, II.b, IV, VII and VIII have the following meaning:
- R¹: is methyl, ethyl, *n*-propyl, *iso*-propyl, *n*-butyl, *sec*-butyl, *iso*-butyl, cyclopropyl, 2-methoxyiminoethyl, bicyclo[1.1.1]pentan-1-yl, or phenyl; and wherein the phenyl group is unsubstituted or substituted with 1, 2, 3 or up to the maximum possible number of identical or different radicals selected from the group consisting of fluorine, chlorine, cyano, OH, NH₂, methyl, ethyl, methoxy, trifluoromethyl, trifluoromethoxy, difluoromethyl, difluoromethoxy, and cyclopropyl; and
- R²: is hydrogen, methyl, or ethyl.

In another preferred embodiment the variables R¹ and R² in compounds of formula I, I.b, II.b, IV, VII and VIII have the following meaning:
- R¹: is methyl, 2-methoxyiminoethyl, bicyclo[1.1.1]pentan-1-yl, 2-fluoro-phenyl, 4-fluoro-phenyl, or 2,4-difluorophenyl; in particular methyl or 2-fluoro-phenyl; and
- R²: is hydrogen.

In the definitions of the variables given above, collective terms are used which are generally representative for the substituents in question.

The term "Cₙ-Cₘ" indicates the number of carbon atoms possible in each case in the substituent or substituent moiety in question.

The term "halogen" refers to fluorine, chlorine, bromine and iodine.

The term "oxo" refers to an oxygen atom =O, which is bound to a carbon atom or sulfur atom, thus forming, for example, a ketonyl -C(=O)- or sulfinyl -S(=O)- group.

The term "C₁-C₆-alkyl" refers to a straight-chained or branched saturated hydrocarbon group having 1 to 6 carbon atoms, for example methyl, ethyl, propyl, 1-methylethyl, butyl, 1-methylpropyl, 2-methylpropyl, and 1,1-dimethylethyl.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "C₁-C₆-alkoxy" refers to a straight-chain or branched alkyl group having 1 to 6 carbon atoms (as defined above) which is bonded via an oxygen, at any position in the alkyl group, for example methoxy, ethoxy, n-propoxy, 1-methylethoxy, butoxy, 1-methylpropoxy, 2-methylpropoxy or 1,1-dimethylethoxy.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to alkyl having 1 to 4 carbon atoms (as defined above), wherein one hydrogen atom of the alkyl radical is replaced by a phenyl or hetereoaryl radical respectively.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₄-alkylthio-C₁-C₄-alkyl" refers to alkyl having 1 to 4 carbon atoms (as defined above), wherein one hydrogen atom of the alkyl radical is replaced by a C₁-C₄-alkylthio group.

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₄-alkoxyimino" refers to a divalent imino radical (C₁-C₄-alkyl-O-N=) carrying one C₁-C₄-alkoxy group as substituent, e.g. methylimino, ethylimino, propylimino, 1-methylethyl-imino, butylimino, 1-methylpropylimino, 2-methylpropylimino, 1,1-dimethylethylimino and the like.

The term "C₁-C₆-alkoxyimino-C₁-C₄-alkyl" refers to alkyl having 1 to 4 carbon atoms, wherein two hydrogen atoms of one carbon atom of the alkyl radical are replaced by a divalent C₁-C₆-alkoxyimino radical (C₁-C₆-alkyl-O-N=) as defined above.

The term "C₂-C₆-alkenyloxyimino-C₁-C₄-alkyl" refers to alkyl having 1 to 4 carbon atoms, wherein two hydrogen atoms of one carbon atom of the alkyl radical are replaced by a divalent C₂-C₆-alkenyloxyimino radical (C₂-C₆-alkenyl-O-N=).

The term "C₂-C₆-alkynyloxyimino-C₁-C₄-alkyl" refers to alkyl having 1 to 4 carbon atoms, wherein two hydrogen atoms of one carbon atom of the alkyl radical are replaced by a divalent C₂-C₆-alkynyloxyimino radical (C₂-C₆-alkynyl-O-N=).

The term "hydroxyC₁-C₄-alkyl" refers to alkyl having 1 to 4 carbon atoms, wherein one hydrogen atom of the alkyl radical is replaced by a OH group.

The term "aminoC₁-C₄-alkyl" refers to alkyl having 1 to 4 carbon atoms, wherein one hydrogen atom of the alkyl radical is replaced by a NH₂ group.

The term "C₁-C₆-alkylamino" refers to an amino group, which is substituted with one residue independently selected from the group that is defined by the term C₁-C₆-alkyl. Likewise, the term "diC₁-C₆-alkylamino" refers to an amino group, which is substituted with two residues independently selected from the group that is defined by the term C₁-C₆-alkyl.

The term "C₁-C₄-alkylamino-C₁-C₄-alkyl" refers to refers to alkyl having 1 to 4 carbon atoms (as defined above), wherein one hydrogen atom of the alkyl radical is replaced by a C₁-C₄-alkyl-NH-group which is bound through the nitrogen. Likewise, the term "diC₁-C₄-alkylamino-C₁-C₄-alkyl" refers to refers to alkyl having 1 to 4 carbon atoms (as defined above), wherein one hydrogen atom of the alkyl radical is replaced by a (C₁-C₄-alkyl)₂N- group which is bound through the nitrogen.

The term "aminocarbonyl-C₁-C₄-alkyl" refers to alkyl having 1 to 4 carbon atoms, wherein one hydrogen atom of the alkyl radical is replaced by a -(C=O)-NH₂ group.

The term "C₃-C₁₄-cycloalkyl" refers to a monocyclic, bicyclic or tricyclic saturated univalent hydrocarbon radical having 3 to 11 carbon ring members that is connected through one of the ring carbon atoms by substitution of one hydrogen atom, such as cyclopropyl (C₃H₅), cyclobutyl, cyclopentyl, cyclohexyl, cycloheptyl, cyclooctyl, bicyclo[1.1.0]butyl, bicyclo[2.1.0]pentyl, bicyclo[1.1.1]pentyl, bicyclo[3.1.0]hexyl, bicyclo[2.1.1]hexyl, norcaranyl (bicyclo[4.1.0]heptyl) and norbornyl (bicyclo[2.2.1]heptyl).

The terms "-C(=O)-C₁-C₆-alkyl", "-C(=O)-O-C₁-C₆-alkyl" and "-C(=O)-C₃-C₁₁-cycloalkyl" refer to aliphatic radicals which are attached through the carbon atom of the -C(=O)- group.

The term "aliphatic" refers to compounds or radicals composed of carbon and hydrogen and which are non-aromatic compounds. An "alicyclic" compound or radical is an organic compound that is both aliphatic and cyclic. They contain one or more all-carbon rings which may be either saturated or unsaturated, but do not have aromatic character.

The terms "cyclic moiety" or "cyclic group" refer to a radical which is an alicyclic ring or an aromatic ring, such as, for example, phenyl or heteroaryl.

The term "and wherein any of the aliphatic or cyclic groups are unsubstituted or substituted with..." refers to aliphatic groups, cyclic groups and groups, which contain an aliphatic and a cyclic moiety in one group, such as in, for example, C₃-C₈-cycloalkyl-C₁-C₄-alkyl; therefore a group which contains an aliphatic and a cyclic moiety both of these moieties may be substituted or unsubstituted independently of each other.

The term "phenyl" refers to an aromatic ring systems incuding six carbon atoms (commonly referred to as benzene ring.

The term "heteroaryl" refers to aromatic monocyclic or polycyclic ring systems incuding besides carbon atoms, 1, 2, 3 or 4 heteroatoms independently selected from the group consisting of N, O and S.

The term "saturated 3- to 7-membered carbocycle" is to be understood as meaning monocyclic saturated carbocycles having 3, 4 or 5 carbon ring members. Examples include cyclopropyl, cyclopentyl, cyclohexyl, cycloheptyl, and the like.

The term "3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms", is to be understood as meaning both, aromatic mono- and bicyclic heteroaromatic ring systems, and also saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine;
and a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6- or-7-yl, 2,3,4,7-tetrahydro[1H]azepin-1-,-2-,-3-,-4-,-5-,-6-or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "5- or 6-membered heteroaryl" or the term "5- or 6-membered aromatic heterocycle" refer to aromatic ring systems incuding besides carbon atoms, 1, 2, 3 or 4 heteroatoms independently selected from the group consisting of N, O and S, for example, a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

### Working Examples

The present invention is further illustrated by means of the following working examples.

Analytical method 1: HPLC Agilent 1100 Series; column: Agilent Zorbax Phenyl-Hexyl 1,8µm 50*4,6mm, Column Flow: 1 mL/min, time: 25 min, pressure: 20000 kPa; temperature: 20°C; wavelength 200 nm; injector volume: 2 uL; retention time of the respective products is based on reference material.

### Eluent: A: Water with 0,1 vol% H₃PO₄; B: Acetonitrile

| Time (min) | %B | Rate (mL/min) |
|---|---|---|
| 0,0 | 14 | 1,0 |
| 16,0 | 86 | 1,0 |
| 20,0 | 86 | 1,0 |
| 20,1 | 14 | 1,0 |

### Example 1) Preparation of N-(2-fluorophenyl)-4-(N'-hydroxycarbamimidoyl)benzamide

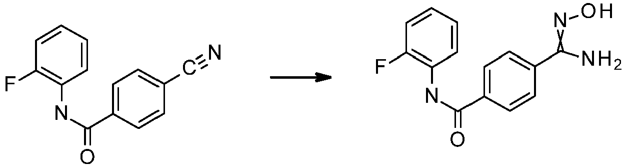

A reaction vessel was charged at room temperature with 816 g methanol (15 mol) and 408.3 g 4-cyano-*N*-(2-fluorophenyl)benzamide (1.7 mol). The reaction mixture was heated to 40°C and then 129.2 g hydroxylamine (1.955 mol, 50% in water) was added within 180 minutes at 40°C. **The resulting suspension was well agitable without further methanol addition.** The mixture was stirred for additional 12 hours at 40°C. Then, 816 g water was added at 40°C within 1 hour. The resulting mixture was cooled to 5°C within 3 hours. The precipitated solid was filtered off, washed with 816 g water and dried under reduced pressure (50 mbar) at 70°C. 467.2 g of product were obtained. (purity: 98.1% (w/w, determined by quantitative HPLC analytics), yield 98.7%).

### Example 2) Preparation of N-(2-fluorophenyl)-4-(N'-hydroxycarbamimidoyl)benzamide

A reaction vessel was charged at room temperature with 118.7 g methanol (3.7 mol), 60.0 g 4-cyano-N-(2-fluorophenyl)benzamide (0.247 mol) and 0.3 g sulfuric acid (98 %, 0.003 mol). The reaction mixture was heated to 40°C and then 18.8 g hydroxylamine (0.284 mol, 50 % in water) was continuously dosed over a time period of 4 hours at 40°C. **The mixture was well agitable over the whole time** (agitator with 600 rpm) and poststirred over 9 hours at 40°C. Then, 170 g water was added at 40°C. The resulting mixture was cooled to room temperature. The precipitated solid was filtered off, washed with 100 g water and dried under reduced pressure (50 mbar) at 80°C. 64.5 g of product were obtained. (purity: 96.9% (w/w, determined by quantitative HPLC analytics), yield 92.5%).

### Example 3) Preparation of N-(2-fluorophenyl)-4-(N'-hydroxycarbamimidoyl)benzamide

A reaction vessel was charged at room temperature with 118.7 g methanol (3.7 mol), 60.0 g 4-cyano-*N*-(2-fluorophenyl)benzamide (0.247 mol) and 0.3 g sulfuric acid (98 %, 0.003 mol). The reaction mixture was heated to 40°C and then 18.8 g hydroxylamine (0.284 mol, 50 % in water) was added within less than 1 minute at 40°C. **The suspension was getting very viscous after 1h and agitation was not feasible anymore** (agitator with 600 rpm did not mix the visible vessel content anymore - such process cannot be scaled up for production). The mixture was nevertheless "poststirred" over 12 hours at 40°C. Then, 170 g water was added at 40°C. The resulting mixture was cooled to room temperature. The precipitated solid was filtered off, washed with 100 g water and dried under reduced pressure (50 mbar) at 80°C. 63.3 g of product were obtained. (purity: 96.7% (w/w, determined by quantitative HPLC analytics), yield 90.6%).

### Example 4) Preparation of N-(2-fluorophenyl)-4-[5-(trifluoromethyl)-1,2,4-oxadiazol-3-yl]benzamide

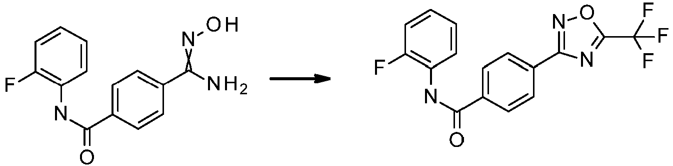

A flask was charged with 500 mg (1.83 mmol) of *N*-(2-fluorophenyl)-4-[(Z)-*N*'-hydroxycarbamimidoyl]benzamide and 5 mL of *N*,*N*-dimethylformamide at room temperature. To this reaction mass 416 mg (2.93 mmol) of ethyl trifluoroacetate was added at room temperature followed by dropwise addition of 527 mg sodium methanolate (2.93 mmol, 30% w/w in methanol). Slight exotherm was observed during this addition and the reaction mass turned reddish brown. The reaction mass was stirred at room temperature for 1 further hour. HPLC analysis confirmed complete conversion. Then, water was added to the reaction mass, which caused the product to precipitate. The product was filtered and the filter cake was washed with water to remove *N*,*N*-dimethylformamide followed by drying to yield 0.53 g (84.6%, purity determined by quantitative HPLC analytics : 97.2%) of the title compound.

## Claims

1. A process for preparing amidoxime compounds of formula I, 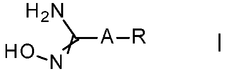 wherein
A is phenyl or a 5- or 6-membered aromatic heterocycle; wherein the ring member atoms of the aromatic heterocycle include besides carbon atoms 1, 2, 3, or 4 heteroatoms selected from N, O, and S as ring member atoms with the provision that the heterocycle cannot contain 2 contiguous atoms selected from O and S; and wherein A is further unsubstituted or further substituted with additional n identical or different radicals R^{A}; wherein
n is 0,1, 2, 3, or 4;
R^{A} is independently selected from the group consisting of halogen, cyano, C₁-C₆-alkyl, C₁-C₆-haloalkyl, C₁-C₆-alkoxy, and C₁-C₆-haloalkoxy;
R is methyl, chloromethyl, hydroxymethyl, trichloromethyl, ethyl, iso-propyl, OH, SH, cyano, halogen, CH₂F, CHF₂, 2,2,2-trifluoroethyl, cyclopropyl,-C(=O)H, -C(=NOR²)H, -C(=O)OH, -C(=O)OR¹, -C(=W)N(R¹R²), -CR³R⁴-N(R¹R²), -CR³R⁴-OR¹, -C(=NR¹)R³, -C(=O)R³, -CR³R⁴-C(=O)OH, -CR³R⁴-C(=O)R¹, -CR³R⁴-C(=W)N(R¹R²), -O-CR³R⁴-C(=O)OH, -O-CR³R⁴-C(=O)R¹, -O-CR³R⁴C(=W)N(R¹R²), -CR³R⁴-N(R²)-C(=W)R¹, -CR³R⁴-S(=O)₂R¹, or -CR³R⁴-N(R²)-S(=O)₂R¹; wherein
W is O or S;
R² is hydrogen, C₁-C₆-alkyl, C₂-C₆-alkenyl, C₂-C₆-alkynyl, C₁-C₆-alkoxy, C₃-C₁₁-cycloalkyl, -C(=O)-C₁-C₆-alkyl, -C(=O)-C₃-C₁₁-cycloalkyl, or -C(=O)-O-C₁-C₆-alkyl; and wherein any of the aliphatic or cyclic groups in R² are unsubstituted or substituted with 1, 2, 3, or up to the maximum possible number of identical or different radicals selected from the group consisting of halogen, hydroxy, oxo, cyano, C₁-C₆-alkyl, C₁-C₆-alkoxy, and C₃-C₁₁-cycloalkyl;
R¹ is C₁-C₆-alkyl, C₁-C₆-alkoxy, C₃-C₁₁-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₆-alkylamino, diC₁-C₆-alkylamino, -C(=O)-C₁-C₆-alkyl, -C(=O)-O-C₁-C₆-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl, or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O, and S as ring member atoms with the provision that the heterocycle cannot contain 2 contiguous atoms selected from O and S; and wherein any of the above-mentioned aliphatic or cyclic groups are unsubstituted or substituted with 1, 2, 3, or up to the maximum possible number of identical or different groups R^{1a}; or
R¹ and R², together with the nitrogen atom to which they are attached, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O, and S as ring member atoms with the provision that the heterocycle cannot contain 2 contiguous atoms selected from O and S; and wherein the heterocycle is unsubstituted or substituted with 1, 2, 3, 4, or up to the maximum possible number of identical or different groups R^{1a}; wherein
R^{1a} is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, -C(=O)-C₁-C₄-alkyl, -C(=O)-O-C₁-C₄-alkyl, C₁-C₆-alkylsulfonyl, hydroxyC₁-C₄-alkyl, -C(=O)-NH₂, -C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl, or C₁-C₄-alkoxy-C₁-C₄-alkyl;
R³, R⁴ independently of each other are selected from the group consisting of hydrogen, halogen, cyano, C₁-C₄-alkyl, C₁-C₄-alkenyl, C₁-C₄-alkynyl, C₁-C₄-haloalkyl and C₁-C₄-alkoxy; or
R³ and R⁴ together with the carbon atom to which they are bound form a cyclopropyl group,
by reacting a nitrile of formula II, wherein the variables A and R are as defined above for compounds of formula I,
N≡C-A-R II
with free hydroxylamine in a solvent comprising an alkyl alcohol of formula III
C₁-C₆-alkyl-OH III,
the process comprising the following steps:
step 1: charging a reaction vessel with a solvent and nitrile II;
step 2: metering 0.9 to 1.5 equivalents of free hydroxylamine, based on the amount of nitrile II, into the mixture obtained in step 1;
whereas the process is **characterized in that** the mixture obtained in step 1 is a suspension containing particulate nitrile II; the free hydroxylamine in step 2 is metered to nitrile II over a period of equal to or more than 15 minutes; and wherein the temperature of the reaction mixture in step 2 of the process is between 20°C to 60°C.

2. The process according to claim 1, wherein the solvent comprises ethanol or methanol.

3. The process according to claim 1 or 2, wherein the temperature of the reaction mixture in step 2 of the process is between 30°C and 50°C.

4. The process according to any one of claims 1 to 3, wherein 1.0 to 1.2 equivalents of free hydroxylamine are added in step 2 to the reaction mixture obtained in step 1.

5. The process according to any one of claims 1 to 4, wherein the reaction mixture obtained in step 1 contains equal to or more than 350 g of nitrile II per kilogram of solvent.

6. The process according to any one of claims 1 to 5, wherein the free hydroxylamine in step 2 is metered to nitrile II over a period of equal to or more than 1 hour.

7. The process according to any one of claims 1 to 6, wherein the nitrile compound is of formula II.b, 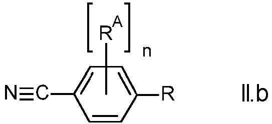 wherein n is 0 or 1, and the meaning of R^{A} and R is as defined in claim 1 for compounds of formula I, to obtain amidoximes of formula I.b, 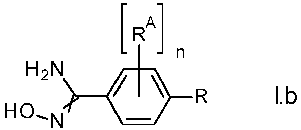 wherein the variables n, R^{A}, and R have the meaning as defined for compounds Il.b.

8. The process according to claim 7, wherein the variables have the following meaning:
R^{A} is fluorine;
n is 0 or 1;
R is methyl, -C(=O)OH, -C(=O)NR¹R², -CH₂-N(R²)-C(=O)R¹, -CH₂-N(R²)-S(=O)₂R¹, 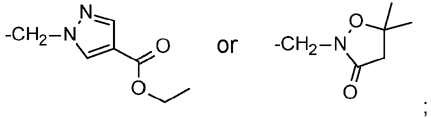
R¹ is C₁-C₆-alkly, phenyl, or cyclopropyl, wherein the phenyl ring is unsubstituted or substituted with 1, 2, 3, or 4 identical or different groups selected from halogen;
R² is hydrogen, methyl, ethyl, methoxy, ethoxy, or cyclopropyl.

9. The process according to claim 7, wherein the variables have the following meaning:
n is 0;
R is -C(=O)N(R¹R²);
R¹ is methyl, 2-methoxyiminoethyl, bicyclo[1.1.1]pentan-1-yl, 2-fluoro-phenyl, 4-fluoro-phenyl, or 2,4-difluorophenyl; in particular methyl or 2-fluoro-phenyl;
R² is hydrogen.

10. The process according to any one of claims 7 to 9, further comprising the step of reacting the compound of formula I.b, to obtain the compound of formula IV.

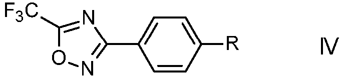

11. The process according to claim 10, wherein n is 0 and R is methyl in compounds of formula IV and further comprising the step of reacting the compound of formula IV to obtain the compound of formula IV.a.

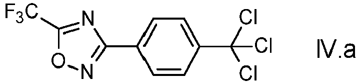

12. The process according to claim 7 or 8, wherein R is -C(=O)OH in compounds of formula I.b and II.b, and further comprising the step of reacting the compound of formula IV to obtain the compound of formula V.

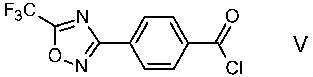

13. The process according to claim 11, further comprising the step of reacting the compound of formula IV.a to obtain the compound of formula V.

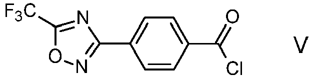

14. The process according to claim 12 or 13, further comprising the step of reacting the compound of formula V with a compound of formula VI,
R¹-NH-R² VI
wherein R¹ and R² in the compound of formula VI is defined as for compounds of formula I in any of the preceding claims to obtain a compound of formula VII.

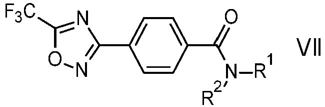

15. The process according to claim 14, further comprising the step of reacting the compound of formula V to obtain a compound of formula VIII.

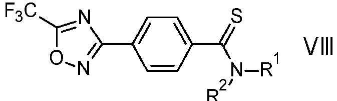

16. The process according to claim 14 or 15, wherein in compounds of formulae VI, VII, and VIII
R¹ is methyl, 2-methoxyiminoethyl, bicyclo[1.1.1]pentan-1-yl, 2-fluoro-phenyl, 4-fluoro-phenyl, or 2,4-difluorophenyl; in particular methyl or 2-fluoro-phenyl; and
R² is hydrogen.

## Patentansprüche

1. Verfahren zur Herstellung von Amidoximverbindungen der Formel I, 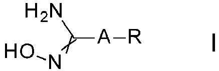 wobei
A Phenyl oder ein 5- oder 6-gliedriger aromatischer Heterocyclus ist; wobei die Ringatome des aromatischen Heterocyclus neben Kohlenstoffatomen 1, 2, 3 oder 4 Heteroatome, ausgewählt aus N, O und S, als Ringatome umfassen, mit der Maßgabe, dass der Heterocyclus nicht 2 benachbarte Atome, ausgewählt aus O und S, enthalten kann; und wobei A ferner unsubstituiert oder ferner mit zusätzlichen n gleichen oder verschiedenen Resten R^{A} substituiert ist; wobei
n 0, 1, 2, 3 oder 4 ist;
R^{A} unabhängig ausgewählt ist aus der Gruppe bestehend aus Halogen, Cyano, C₁-C₆-Alkyl, C₁-C₆-Halogenalkyl, C₁-C₆-Alkoxy und C₁-C₆-Halogenalkoxy;
R Methyl, Chloromethyl, Hydroxymethyl, Trichlormethyl, Ethyl, iso-Propyl, OH, SH, Cyano, Halogen, CH₂F, CHF₂, 2,2,2-Trifluorethyl, Cyclopropyl, -C(=O)H, C(=NOR²)H, -C(=O)OH, -C(=O)OR¹, -C(=W)N(R¹R²), CR³R⁴-N(R¹R²), CR³R⁴-OR¹, C(=NR¹)R³, C(=O)R³, -CR³R⁴-C(=O)OH, CR³R⁴-C(=O)R¹, CR³R⁴-C(=W)N(R¹R²), O-CR³R⁴-C(=O)OH, -O-CR³R⁴-C(=O)R¹, O-CR³R⁴C(=W)N(R¹R²), CR³R⁴-N(R²)-C(=W)R¹, CR³R⁴-S(=O)₂R¹ oder CR³R⁴-N(R²)-S(=O)₂R¹ ist; wobei
W O oder S ist;
R² Wasserstoff, C₁-C₆-Alkyl, C₂-C₆-Alkenyl, C₂-C₆-Alkinyl, C₁-C₆-Alkoxy, C₃-C₁₁-Cycloalkyl, C(=O)-C₁-C₆-Alkyl, -C(=O)-C₃-C₁₁-Cycloalkyl oder -C(=O)O-C₁-C₆-Alkyl ist; und wobei jede der aliphatischen oder cyclischen Gruppen in R² unsubstituiert oder mit 1, 2, 3 oder bis zur maximal möglichen Anzahl gleicher oder verschiedener Reste substituiert ist, ausgewählt aus der Gruppe bestehend aus Halogen, Hydroxy, Oxo, Cyano, C₁-C₆-Alkyl, C₁-C₆-Alkoxy und C₃-C₁₁-Cycloalkyl;
R¹ C₁-C₆-Alkyl, C₁-C₆-Alkoxy, C₃-C₁₁-Cycloalkyl, C₃-C₈-Cycloalkenyl, C₂-C₆-Alkenyl, C₂-C₆-Alkinyl, C₁-C₆-Alkoxyimino-C₁-C₄-Alkyl, C₂-C₆-Alkenyloxyimino-C₁-C₄-Alkyl, C₂-C₆-Alkinyloxyimino-C₁-C₄-Alkyl, C₁-C₆-Alkylamino, diC₁-C₆-Alkylamino, -C(=O)-C₁-C₆-Alkyl, -C(=O)O-C₁-C₆-Alkyl, Phenyl-C₁-C₄-Alkyl, Phenyl-C₁-C₄-Alkenyl, Phenyl-C₁-C₄-Alkinyl, Heteroaryl-C₁-C₄-Alkyl, Phenyl, Naphthyl oder ein 3-bis 10-gliedriger gesättigter, teilweise ungesättigter oder aromatischer mono- oder bicyclischer Heterocyclus ist, wobei die Ringatome des mono- oder bicyclischen Heterocyclus neben Kohlenstoffatomen ferner 1, 2, 3 oder 4 Heteroatome, ausgewählt aus N, O und S, als Ringatome umfassen, mit der Maßgabe, dass der Heterocyclus nicht 2 benachbarte Atome, ausgewählt aus O und S, enthalten kann; und wobei die Heteroarylgruppe in der Gruppe Heteroaryl-C₁-C₄-Alkyl ein 5- oder 6-gliedriger aromatischer Heterocyclus ist, wobei die Ringatome des heterocyclischen Rings neben Kohlenstoffatomen 1, 2, 3 oder 4 Heteroatome, ausgewählt aus N, O und S, als Ringatome umfassen, mit der Maßgabe, dass der Heterocyclus nicht 2 benachbarte Atome, ausgewählt aus O und S, enthalten kann; und wobei jede der vorstehend genannten aliphatischen oder cyclischen Gruppen unsubstituiert oder mit 1, 2, 3 oder bis zur maximal möglichen Anzahl gleicher oder verschiedener Gruppen R^{1a} substituiert ist; oder
R¹ und R² zusammen mit dem Stickstoffatom, an das sie gebunden sind, einen gesättigten oder teilweise ungesättigten mono- oder bicyclischen 3- bis 10-gliedrigen Heterocyclus bilden, wobei der Heterocyclus neben einem Stickstoffatom und einem oder mehreren Kohlenstoffatomen keine weiteren Heteroatome oder 1, 2 oder 3 weitere Heteroatome, unabhängig ausgewählt aus N, O und S, als Ringatome umfasst, mit der Maßgabe, dass der Heterocyclus nicht 2 benachbarte Atome, ausgewählt aus O und S, enthalten kann; und wobei der Heterocyclus unsubstituiert oder mit 1, 2, 3, 4 oder bis zur maximal möglichen Anzahl gleicher oder verschiedener Gruppen R^{1a} substituiert ist; wobei
R^{1a} Halogen, Oxo, Cyano, NO₂, OH, SH, NH₂, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, NHSO₂C₁-C₄-Alkyl, -C(=O)-C₁-C₄-Alkyl, -C(=O)-O-C₁-C₄-Alkyl, C₁-C₆-Alkylsulfonyl, HydroxyC₁-C₄-Alkyl, -C(=O)-NH₂, -C(=O)-NH(C₁-C₄-Alkyl), C₁-C₄-Alkylthio-C₁-C₄-Alkyl, AminoC₁-C₄-Alkyl, C₁-C₄-Alkylamino-C₁-C₄-Alkyl, diC₁-C₄-Alkylamino-C₁-C₄-Alkyl, Aminocarbonyl-C₁-C₄-Alkyl oder C₁-C₄-Alkoxy-C₁-C₄-Alkyl ist;
R³, R⁴ unabhängig voneinander ausgewählt sind aus der Gruppe bestehend aus Wasserstoff, Halogen, Cyano, C₁-C₄-Alkyl, C₁-C₄-Alkenyl, C₁-C₄-Alkinyl, C₁-C₄-Halogenalkyl und C₁-C₄-Alkoxy; oder
R³ und R⁴ zusammen mit dem Kohlenstoffatom, an das sie gebunden sind, eine Cyclopropylgruppe bilden,
durch Umsetzen eines Nitrils der Formel II, wobei die Variablen A und R wie vorstehend für Verbindungen der Formel I definiert sind,
N≡C-A-R II
mit freiem Hydroxylamin in einem Lösungsmittel, umfassend einen Alkylalkohol der Formel III
C₁-C₆-Alkyl-OH III,
wobei das Verfahren die folgenden Schritte umfasst:
Schritt 1: Beschicken eines Reaktionsgefäßes mit einem Lösungsmittel und Nitril II;
Schritt 2: Zudosieren von 0,9 bis 1,5 Äquivalenten freiem Hydroxylamin, bezogen auf die Menge an Nitril II, zu der in Schritt 1 erhaltenen Mischung;
wobei das Verfahren **dadurch gekennzeichnet ist, dass** die in Schritt 1 erhaltene Mischung eine Suspension ist, die teilchenförmiges Nitril II enthält; das freie Hydroxylamin in Schritt 2 über einen Zeitraum von gleich oder mehr als 15 Minuten zu Nitril II zudosiert wird; und wobei die Temperatur der Reaktionsmischung in Schritt 2 des Verfahrens zwischen 20°C und 60°C liegt.

2. Das Verfahren nach Anspruch 1, wobei das Lösungsmittel Ethanol oder Methanol umfasst.

3. Das Verfahren nach Anspruch 1 oder 2, wobei die Temperatur der Reaktionsmischung in Schritt 2 des Verfahrens zwischen 30°C und 50°C liegt.

4. Das Verfahren nach einem der Ansprüche 1 bis 3, wobei in Schritt 2 1,0 bis 1,2 Äquivalente freies Hydroxylamin zu der in Schritt 1 erhaltenen Reaktionsmischung gegeben werden.

5. Das Verfahren nach einem der Ansprüche 1 bis 4, wobei die in Schritt 1 erhaltene Reaktionsmischung gleich oder mehr als 350 g Nitril II pro Kilogramm Lösungsmittel enthält.

6. Das Verfahren nach einem der Ansprüche 1 bis 5, wobei das freie Hydroxylamin in Schritt 2 über einen Zeitraum von gleich oder mehr als 1 Stunde zu Nitril II zudosiert wird.

7. Das Verfahren nach einem der Ansprüche 1 bis 6, wobei die Nitrilverbindung der Formel Il.b entspricht, 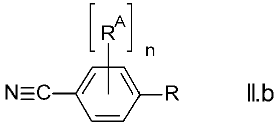 wobei n 0 oder 1 ist und die Bedeutung von R^{A} und R wie in Anspruch 1 für Verbindungen der Formel I definiert ist, um Amidoxime der Formel I.b zu erhalten, 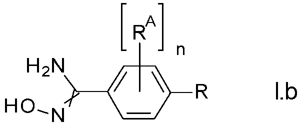 wobei die Variablen n, R^{A} und R die für Verbindungen Il.b definierte Bedeutung haben.

8. Das Verfahren nach Anspruch 7, wobei die Variablen die folgende Bedeutung haben:
R^{A} Fluor ist;
n 0 oder 1 ist;
R Methyl, -C(=O)OH, -C(=O)NR¹R², CH₂-N(R²)-C(=O)R¹, CH₂-N(R²)-S(=O)₂R¹ ist;
R¹ C₁-C₆-Alkyl, Phenyl oder Cyclopropyl ist, wobei der Phenylring unsubstituiert oder mit 1, 2, 3 oder 4 gleichen oder verschiedenen Gruppen, ausgewählt aus Halogen, substituiert ist;
R² Wasserstoff, Methyl, Ethyl, Methoxy, Ethoxy oder Cyclopropyl ist.

9. Das Verfahren nach Anspruch 7, wobei die Variablen die folgende Bedeutung haben:
n 0 ist;
R -C(=O)N(R¹R²) ist;
R¹ Methyl, 2-Methoxyiminoethyl, Bicyclo[1.1.1]pentan-1-yl, 2-Fluorphenyl, 4-Fluorphenyl oder 2,4-Difluorphenyl ist; insbesondere Methyl oder 2-Fluorphenyl;
R² Wasserstoff ist.

10. Das Verfahren nach einem der Ansprüche 7 bis 9, ferner umfassend den Schritt des Umsetzens der Verbindung der Formel I.b, um die Verbindung der Formel IV zu erhalten.

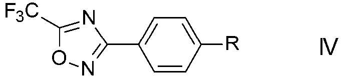

11. Das Verfahren nach Anspruch 10, wobei n 0 ist und R Methyl in Verbindungen der Formel IV ist, und ferner umfassend den Schritt des Umsetzens der Verbindung der Formel IV, um die Verbindung der Formel IV.a zu erhalten.

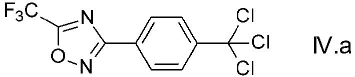

12. Das Verfahren nach Anspruch 7 oder 8, wobei R -C(=O)OH in Verbindungen der Formeln I.b und II.b ist, und ferner umfassend den Schritt des Umsetzens der Verbindung der Formel IV, um die Verbindung der Formel V zu erhalten.

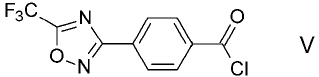

13. Das Verfahren nach Anspruch 11, ferner umfassend den Schritt des Umsetzens der Verbindung der Formel IV.a, um die Verbindung der Formel V zu erhalten.

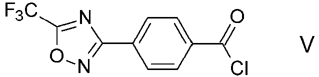

14. Das Verfahren nach Anspruch 12 oder 13, ferner umfassend den Schritt des Umsetzens der Verbindung der Formel V mit einer Verbindung der Formel VI,
R¹-NH-R² VI
wobei R¹ und R² in der Verbindung der Formel VI wie für Verbindungen der Formel I in einem der vorstehenden Ansprüche definiert sind, um eine Verbindung der Formel VII zu erhalten.

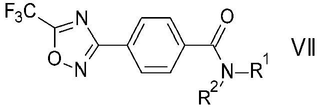

15. Das Verfahren nach Anspruch 14, ferner umfassend den Schritt des Umsetzens der Verbindung der Formel V, um eine Verbindung der Formel VIII zu erhalten.

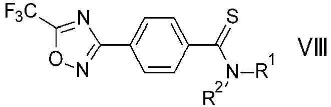

16. Das Verfahren nach Anspruch 14 oder 15, wobei in Verbindungen der Formeln VI, VII und VIII
R¹ Methyl, 2-Methoxyiminoethyl, Bicyclo[1.1.1]pentan-1-yl, 2-Fluorphenyl, 4-Fluorphenyl oder 2,4-Difluorphenyl ist; insbesondere Methyl oder 2-Fluorphenyl; und
R² Wasserstoff ist.

## Revendications

1. Un procédé de préparation de composés amidoxime de formule I, 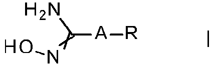 dans lequel
A est un phényle ou un hétérocycle aromatique à 5 ou 6 chaînons ; dans lequel les atomes membres du cycle de l'hétérocycle aromatique comprennent, outre des atomes de carbone, 1, 2, 3 ou 4 hétéroatomes choisis parmi N, O et S en tant qu'atomes membres du cycle, à condition que l'hétérocycle ne puisse pas contenir 2 atomes contigus choisis parmi O et S ; et dans lequel A est en outre non substitué ou en outre substitué par n radicaux identiques ou différents supplémentaires R^{A} ; dans lequel
n est 0, 1, 2, 3 ou 4 ;
R^{A} R^{A} est indépendamment choisi dans le groupe constitué par halogène, cyano, C₁-C₆-alkyle, C₁-C₆-haloalkyle, C₁-C₆-alcoxy et C₁-C₆-haloalcoxy ;
R est méthyle, chlorométhyle, hydroxyméthyle, trichlorométhyle, éthyle, iso-propyle, OH, SH, cyano, halogène, CH₂F, CHF₂, 2,2,2-trifluoroéthyle, cyclopropyle, -C(=O)H, C(=NOR²)H, -C(=O)OH, -C(=O)OR¹, -C(=W)N(R¹R²), CR³R⁴-N(R¹R²), CR³R⁴-OR¹, C(=NR¹)R³, C(=O)R³, -CR³R⁴-C(=O)OH, CR³R⁴-C(=O)R¹, CR³R⁴-C(=W)N(R¹R²), O-CR³R⁴-C(=O)OH, -O-CR³R⁴-C(=O)R¹, O-CR³R⁴C(=W)N(R¹R²), CR³R⁴-N(R²)-C(=W)R¹, CR³R⁴-S(=O)₂R¹ ou CR³R⁴-N(R²)-S(=O)₂R¹ ; dans lequel
W est O ou S ;
R² est hydrogène, C₁-C₆-alkyle, C₂C₆-alcényle, C₂C₆-alcynyle, C₁-C₆-alcoxy, C₃C₁₁cycloalkyle, C(=O)-C₁C₆-alkyle, -C(=O)-C₃C₁₁-Cycloalkyle ou -C(=O)O-C₁C₆-alkyle ; et dans lequel l'un quelconque des groupes aliphatiques ou cycliques dans R² est non substitué ou substitué par 1, 2, 3 ou jusqu'au nombre maximum possible de radicaux identiques ou différents choisis dans le groupe constitué par halogène, hydroxy, oxo, cyano, C₁C₆alkyle, C₁C₆-alcoxy et C₃-C₁₁-cycloalkyle ;
R¹ est C₁-C₆-alkyle, C₁-C₆-alcoxy, C₃C₁₁-cycloalkyle, C₃C₈cycloalcényle, C₂C₆-alcényle, C₂C₆-alcynyle, C₁-C₆-alcoxyimino-C₁-C₄-alkyle, C₂-C₆-alcényloxyimino-C₁-C₄-alkyle, C₂C₆-alcynyloxyimino-C₁-C₄-alkyle, C₁-C₆-alkylamino, diC₁-C₆-alkylamino, -C(=O)-C₁C₆-alkyle, -C(=O)O-C₁C₆-alkyle, phényl-C₁C₄alkyle, phényl-C₁C₄alcényle, phényl-C₁C₄alcynyle, hétéroaryl-C₁-C₄-alkyle, phényle, naphtyle ou un hétérocycle mono-ou bicyclique saturé, partiellement insaturé ou aromatique à 3 à 10 chaînons, dans lequel les atomes membres du cycle dudit hétérocycle mono- ou bicyclique comprennent, outre des atomes de carbone, 1, 2, 3 ou 4 hétéroatomes supplémentaires choisis parmi N, O et S en tant qu'atomes membres du cycle, à condition que l'hétérocycle ne puisse pas contenir 2 atomes contigus choisis parmi O et S ; et dans lequel le groupe hétéroaryle dans le groupe hétéroaryl-C₁-C₄-alkyle est un hétérocycle aromatique à 5 ou 6 chaînons, dans lequel les atomes membres du cycle hétérocyclique comprennent, outre des atomes de carbone, 1, 2, 3 ou 4 hétéroatomes choisis parmi N, O et S en tant qu'atomes membres du cycle, à condition que l'hétérocycle ne puisse pas contenir 2 atomes contigus choisis parmi O et S ; et dans lequel l'un quelconque des groupes aliphatiques ou cycliques mentionnés ci-dessus est non substitué ou substitué par 1, 2, 3 ou jusqu'au nombre maximum possible de groupes identiques ou différents R^{1a} ; ou
R¹ et R², conjointement avec l'atome d'azote auquel ils sont attachés, forment un hétérocycle mono- ou bicyclique saturé ou partiellement insaturé à 3 à 10 chaînons, dans lequel l'hétérocycle comprend, outre un atome d'azote et un ou plusieurs atomes de carbone, aucun autre hétéroatome ou 1, 2 ou 3 hétéroatomes supplémentaires indépendamment choisis parmi N, O et S en tant qu'atomes membres du cycle, à condition que l'hétérocycle ne puisse pas contenir 2 atomes contigus choisis parmi O et S ; et dans lequel l'hétérocycle est non substitué ou substitué par 1, 2, 3, 4 ou jusqu'au nombre maximum possible de groupes identiques ou différents R^{1a} ; dans lequel
R^{1a} est halogène, oxo, cyano, NO₂, OH, SH, NH₂, C₁C₆alkyle, C₁C₆haloalkyle, C₁C₆alcoxy, C₁C₆haloalcoxy, C₁C₆-alkylthio, C₁-C₆-haloalkylthio, C₃C₈cycloalkyle, NHSO₂C₁C₄-alkyle, -C(=O)-C₁C₄-alkyle, -C(=O)-O-C₁C₄-alkyle, C₁C₆-alkylsulfonyle, hydroxyC₁-C₄-alkyle, -C(=O)-NH₂, -C(=O)-NH(C₁C₄-alkyle), C₁-C₄-alkylthio-C₁-C₄-alkyle, aminoC₁-C₄-alkyle, C₁C₄-alkylamino-C₁-C₄-alkyle, diC₁-C₄-alkylamino-C₁-C₄-alkyle, aminocarbonyl-C₁-C₄-alkyle ou C₁-C₄-alcoxy-C₁C₄-alkyle ;
R³, R⁴ indépendamment l'un de l'autre sont choisis dans le groupe constitué par hydrogène, halogène, cyano, C₁C₄alkyle, C₁C₄alcényle, C₁C₄alcynyle, C₁C₄haloalkyle et C₁C₄alcoxy ; ou
R³ et R⁴ conjointement avec l'atome de carbone auquel ils sont liés forment un groupe cyclopropyle,
par réaction d'un nitrile de formule II, dans lequel les variables A et R sont telles que définies ci-dessus pour les composés de formule I,
N≡C-A-R Il
avec de l'hydroxylamine libre dans un solvant comprenant un alcool alkylique de formule III
C₁-C₆-alkyl-OH III,
le procédé comprenant les étapes suivantes :
étape 1 : chargement d'un récipient réactionnel avec un solvant et le nitrile II ;
étape 2 : dosage de 0,9 à 1,5 équivalents d'hydroxylamine libre, par rapport à la quantité de nitrile II, dans le mélange obtenu à l'étape 1 ;
tandis que le procédé est **caractérisé en ce que** le mélange obtenu à l'étape 1 est une suspension contenant du nitrile II particulaire ; l'hydroxylamine libre à l'étape 2 est dosée au nitrile II sur une période égale ou supérieure à 15 minutes ; et dans lequel la température du mélange réactionnel à l'étape 2 du procédé est comprise entre 20 °C et 60 °C.

2. Le procédé selon la revendication 1, dans lequel le solvant comprend de l'éthanol ou du méthanol.

3. Le procédé selon la revendication 1 ou 2, dans lequel la température du mélange réactionnel à l'étape 2 du procédé est comprise entre 30 °C et 50 °C.

4. Le procédé selon l'une quelconque des revendications 1 à 3, dans lequel 1,0 à 1,2 équivalents d'hydroxylamine libre sont ajoutés à l'étape 2 au mélange réactionnel obtenu à l'étape 1.

5. Le procédé selon l'une quelconque des revendications 1 à 4, dans lequel le mélange réactionnel obtenu à l'étape 1 contient au moins 350 g de nitrile II par kilogramme de solvant.

6. Le procédé selon l'une quelconque des revendications 1 à 5, dans lequel l'hydroxylamine libre à l'étape 2 est dosée au nitrile II sur une période égale ou supérieure à 1 heure.

7. Le procédé selon l'une quelconque des revendications 1 à 6, dans lequel le composé nitrile est de formule Il.b, 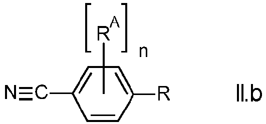 dans lequel n est 0 ou 1, et la signification de R^{A} et R est telle que définie dans la revendication 1 pour les composés de formule I, pour obtenir des amidoximes de formule I.b, 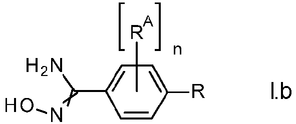 dans lequel les variables n, R^{A} et R ont la signification telle que définie pour les composés Il.b.

8. Le procédé selon la revendication 7, dans lequel les variables ont la signification suivante :
R^{A} est fluor ;
n est 0 ou 1 ;
R est méthyle, -C(=O)OH, -C(=O)NR --¹R², CH₂-N(R²)-C(=O)R¹, CH₂-N(R²)-S(=O)₂R¹ 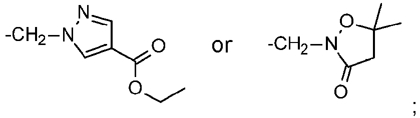
R¹ est C₁-C₆-alkyle, phényle ou cyclopropyle, dans lequel le cycle phényle est non substitué ou substitué par 1, 2, 3 ou 4 groupes identiques ou différents choisis parmi halogène ;
R² est hydrogène, méthyle, éthyle, méthoxy, éthoxy ou cyclopropyle.

9. Le procédé selon la revendication 7, dans lequel les variables ont la signification suivante :
n est 0 ;
R est -C(=O)N(R¹R²) ;
R¹ est méthyle, 2-méthoxyiminoéthyle, bicyclo[1.1.1]pentan-1-yle, 2-fluoro-phényle, 4-fluoro-phényle ou 2,4-difluorophényle ; en particulier méthyle ou 2-fluoro-phényle ;
R² est hydrogène.

10. Le procédé selon l'une quelconque des revendications 7 à 9, comprenant en outre l'étape de réaction du composé de formule I.b, pour obtenir le composé de formule IV.

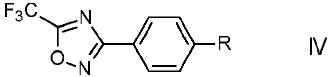

11. Le procédé selon la revendication 10, dans lequel n est 0 et R est méthyle dans les composés de formule IV et comprenant en outre l'étape de réaction du composé de formule IV pour obtenir le composé de formule IV.a.

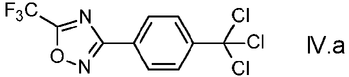

12. Le procédé selon la revendication 7 ou 8, dans lequel R est -C(=O)OH dans les composés de formule I.b et Il.b, et comprenant en outre l'étape de réaction du composé de formule IV pour obtenir le composé de formule V.

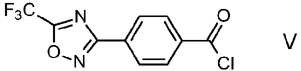

13. Le procédé selon la revendication 11, comprenant en outre l'étape de réaction du composé de formule IV.a pour obtenir le composé de formule V.

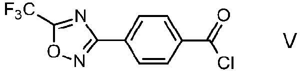

14. Le procédé selon la revendication 12 ou 13, comprenant en outre l'étape de réaction du composé de formule V avec un composé de formule VI,
R¹-NH-R² VI
dans lequel R¹ et R² dans le composé de formule VI sont définis comme pour les composés de formule I dans l'une quelconque des revendications précédentes pour obtenir un composé de formule VII.

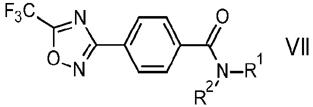

15. Le procédé selon la revendication 14, comprenant en outre l'étape de réaction du composé de formule V pour obtenir un composé de formule VIII.

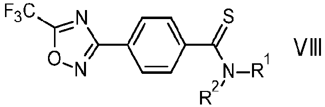

16. Le procédé selon la revendication 14 ou 15, dans lequel dans les composés de formules VI, VII et VIII
R¹ est méthyle, 2-méthoxyiminoéthyle, bicyclo[1.1.1]pentan-1-yle, 2-fluoro-phényle, 4-fluoro-phényle ou 2,4-difluorophényle ; en particulier méthyle ou 2-fluoro-phényle ; et
R ²est hydrogène.
